# EUROPEAN PATENT SPECIFICATION

(11) **EP 4 731 090 B1**
(45) Date of publication and mention of the grant of the patent: **07.10.2026**
(21) Application number: 24734874.1
(22) Date of filing: 18.06.2024
(51) Int. Cl.: A61B 8/00

(54) **SYSTEM FOR MONITORING A CABLE**
SYSTEM ZUR ÜBERWACHUNG EINES ULTRASCHALLSONDENKABELS
SYSTÈME DE SURVEILLANCE DE CÂBLE DE SONDE À ULTRASONS

(30) Priority: 26.06.2023 US 202363523265 P; 17.08.2023 EP 23191985
(43) Date of publication of application: 29.04.2026
(73) Proprietor: Koninklijke Philips N.V., 5656 AE Eindhoven (NL)
(72) Inventor: PERRONE, Antonio Luigi, 5656 AG Eindhoven (NL); MERAL, Faik Can, 5656 AG Eindhoven (NL); SHI, William Tao, 5656 AG Eindhoven (NL); PREVRHAL, Sven Peter, 5656 AG Eindhoven (NL); YANEZ MORENO, Nicolas, 5656 AG Eindhoven (NL); YOUNIS, Khaled Salem Abdalleh, 5656 AG Eindhoven (NL)
(74) Representative: Philips Intellectual Property & Standards
(86) International application number: PCT/EP2024/066871
(87) International publication number: WO 2025/002898

(56) References cited:
- US-A1- 2012 082 422
- US-A1- 2013 131 499
- LINA M CASTANO ET AL: "Smart fabric sensors and e-textile technologies: a review", SMART MATERIALS AND STRUCTURES, vol. 23, no. 5, 1 April 2014 (2014-04-01), GB, pages 053001, XP055558922, ISSN: 0964-1726, DOI: 10.1088/0964-1726/23/5/053001

## Description

### FIELD

The invention relates to predicting ultrasound cable integrity and/or forecasting a remaining useful life of ultrasound cables, an ultrasound cable connecting an ultrasound probe to an ultrasound host comprising the computing device), and an ultrasound system comprising the ultrasound probe, the ultrasound cable, and the ultrasound host.

### BACKGROUND

US 2012/0082422 A1 discloses an electric cable that includes a strain sensor longitudinally extending along the cable.

Castano et al. (2014) "smart fabric sensors and e-textile technologies: a review" discloses a review of smart fabric sensors and electronic textile technologies.

US 2013/0131499 A1 discloses an optical fiber for shape sensing of a cable connected to a transducer device.

Ultrasound (US) examination is a common imaging medium in many medical fields. A US imaging system includes a US probe comprising an acoustic transducer assembly used to send and receive ultrasound signals, which are then converted to electrical signals that are processed to provide images of a region of a body being examined.

The US probe is tethered to a workstation by a US cable, which is used for transmission of electrical and US signals between the US probe and the workstation, as well as power transmissions, for example. US cables are expensive and have a limited useful life, after which their performance hinders the quality of imaging provided by the US imaging system. Known US cables have varying lengths, contents, and compositions, but share common features of comparatively poor elasticity and extensibility. During use, a probe cable is subject to a variety of forces caused by manipulation of the US probe to which it is attached when acquiring desired images during an examination. These forces can vary in magnitude, but over time not only can result in wear and damage to a US cable, but also can reduce the performance of the US cable.

The strain and wear on US cables are thus responsible for a significant percentage of all the failures registered in the radiology department of a hospital. Ultimately, this wear and damage result in the need to replace the US cable. Down time for maintenance of a US machine is exacted as a cost for the hospital that is beneficially reduced or eliminated.

What is needed, therefore, is a system for identifying undue wear, or damage, or both, of the US cable that addresses at least the drawbacks of known systems described above.

### SUMMARY

The invention is defined by the independent claims. The dependent claims define advantageous embodiments.

A first aspect of the invention provides an ultrasound host according to claim 1.

In this manner, through the strain detected along the cable, the host can determine whether the cable is strained beyond its design specifications. Thereby, a compromised cable (e.g., the cable integrity) can be identified and/or the remaining life of the cable can be determined. Collecting strain information over time can further assist in this task as repeated bending, pinching and other motion of the cable may affect the integrity and/or remaining life of the cable. While each individual motion may not exceed design specifications, the repetition of certain motions (e.g., bending) can also lead to material fatigue and eventual breakage. In other words, the variations of strain over time that lead to material fatigue may be detected based on which the cable integrity and remaining useful life can be determined.

An embodiment further comprises any one or a combination of processing the signal to determine an actual three-dimensional shape of the cable; processing the signal to determine a force acting along the length of the cable; and wherein the predicted integrity and/or the forecasted remaining useful life is further based on the actual three-dimensional shape of the cable and/or the force acting along the length of the cable.

From the strain in the cable, it is possible to determine the shape of the cable. The shape can be used as another indicator, for example, for cable pinching.

An embodiment further comprises determining any one or a combination of a position of a clinician handling an ultrasound probe connected to one end of the cable; movements of the clinician operating the ultrasound probe; a three-dimensional position of the ultrasound probe based on a three-dimensional shape of the ultrasound cable and a three-dimensional position of a host connected to another end of the cable; and a three-dimensional surface anatomy based on positions of the ultrasound probe.

Based on the shape of the cable, in ultrasound examinations, it is possible to determine, based on knowing the cable connection with a host or cart, the location of the ultrasound probe. This information can further be used to predict the cable integrity and/or forecast the remaining life of the cable.

An embodiment further comprises processing the signal to determine a location of compromised integrity of the cable, and outputting the location of compromised integrity of the cable.

In this manner, it is for example possible to identify repeated breaking points and improve designs of future cables. Further, it may be possible to reinforce locations of potentially compromised integrity, or soon to be compromised.

An embodiment further comprises issuing, based on the cable integrity and/or the remaining useful life of the cable, a notification to replace the cable.

In this way a user can be alerted well in time to schedule maintenance or replacement of cables, improving planning efficiency and potentially down-time of machines hooked up to the cables.

In an embodiment, the processing and/or determining steps are performed by one or more trained machine learning models.

An embodiment further comprises receiving a motion indication of the cable, responsive to which the method is executed.

A second aspect of the invention provides an ultrasound cable according to claim 7.

Resistive strain sensors in textiles offer cheap alternatives to conventional optical sensors or other known sensors that can be used to infer strain information of cables. Furthermore, the textile substrate may be placed over the cable in the form of a sleeve, such that the sensor can be replaced or moved to a different cable.

In an embodiment of the cable, the resistive strain sensor comprises an electronic textile, E-textile, fabric positioned along the length of the cable.

In an embodiment of the cable, the E-textile fabric comprises a first element along the length of the cable and a second element positioned perpendicularly to the first element.

By providing two different orientations along the cable, it is possible to determine strain, and thus forces and the shape of the cable in different spatial directions. Thereby potentially enabling full three-dimensional shape sensing.

In an embodiment of the cable, the E-textile fabric is disposed helicoidally around the cable.

A helicoidal shape of the sensor or the textile substrate holding the sensor allows for a single distributed sensor to collect strain data relating to different spatial directions. Thereby potentially enabling full three-dimensional shape sensing.

In an embodiment of the cable, the sensor is along a substantial length of the cable, preferably more than 50% of the total length of the cable.

In this way a relevant length of the cable, that is a length of the cable that typically moves, can be monitored.

An embodiment of the cable further comprises a motion sensor.

Another aspect of the invention provides an ultrasound system comprising a host as disclosed herein; an ultrasound probe; and a cable as disclosed herein.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 is a simplified block diagram of a system for imaging a portion of a body, according to a representative embodiment.
Fig. 2 is a perspective view of a US cable comprising a sensor disposed thereover, according to a representative embodiment.
Fig. 3 is a perspective view of a US cable comprising a sensor disposed thereover, according to a representative embodiment.
Fig. 4A is a perspective view of a US cable comprising a sensor disposed thereover, according to a representative embodiment.
Fig. 4B is another perspective view of a US cable comprising a sensor disposed thereover, according to a representative embodiment.
Fig. 4C is a perspective view of a US cable comprising a sensor disposed thereover, according to a representative embodiment.
Fig. 4D is a perspective view of a US cable comprising a sensor disposed thereover, according to a representative embodiment.
Fig. 5A is a simplified flow diagram showing a method of training a computational model for determining a type and an amount of physical change in a US cable according to a representative embodiment.
Fig. 5B is a simplified flow diagram of a computational model determining a type and amount of physical change in a US cable, in accordance with a representative embodiment of the representative embodiments.
Fig. 6A is a simplified flow diagram showing a method of training a computational model for determining a remaining useful life (RUL) in a US cable according to a representative embodiment.
Fig. 6B is a simplified flow diagram of a computational model determining an RUL of a US cable, in accordance with a representative embodiment of the representative embodiments.
Fig. 7A is a simplified flow diagram showing a method of training a computational model for determining an actual shape of the US cable according to a representative embodiment.
Fig. 7B is a simplified flow diagram of a computational model determining an actual shape of a US cable, in accordance with a representative embodiment of the representative embodiments.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials, and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for the purpose of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure. In particular, the description focuses on an ultrasound cable connecting an ultrasound probe with a host or cart. However, it should be understood by a person skilled in the art that the teachings expand to any type of cable configured for data or power transmission.

By the present teachings, a system for monitoring various aspects of a cable used in medical imaging is described. As discussed more fully below, a sensor gathers data related to the location and shape of, and forces acting on a US cable. The sensors are adapted to passively monitor the movement, shape, and forces action on the US cable. These data are then input to a trained computational model to infer one or more of a number of useful information.

As described more fully in connection with representative embodiment described below, the application of a trained computational model enables a use to predict cable integrity of the US cable based on ground truth data, or to forecast a remaining useful life (RUL) of the US cable using ground truth data, or to determine an actual shape of the US cable, or a combination thereof. As will be appreciated by one of ordinary skill in the art, among other possible uses and benefits, the system of the present teachings includes monitoring of wear of the US cable, thereby warning of the need for preventive maintenance. The application of trained computational models also aids in the identification of locations where damage typically occurs to the cable during use (e.g., falling under the cart's wheels, presence of bending or knots that could endanger or limit its integrity etc.) thereby providing an alert to the sonographer of the precarious position of the US cable. Ultimately, the systems of the various representative embodiments usefully forecast or prevent down-times for the machines. By avoiding unscheduled down time and premature wear to the US cable, and among other benefits, the present teachings foster economic benefits for the hospital or clinic using the US machine.

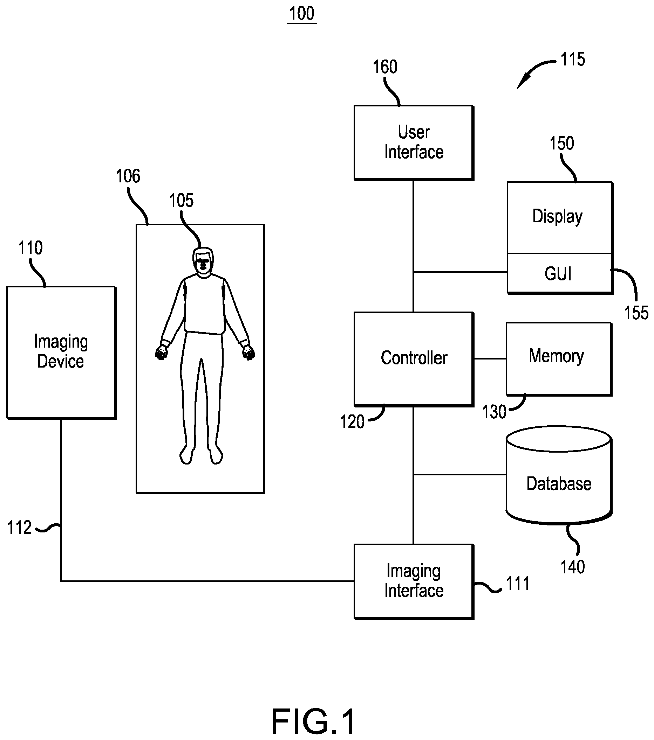

Fig. 1 is a simplified block diagram of a system 100 for imaging a region of interest of a subject, according to a representative embodiment.

Referring to Fig. 1, the system 100 includes an imaging device 110 and a computer system 115 for controlling imaging of a region of interest in a patient 105 on a table 106. The imaging device 110 may be any type of medical imaging device capable of providing an image scan of the region of interest in the patient 105. In accordance with representative embodiments described below, the imaging device 110 may be a US probe or other imaging modality compatible with the methods and systems of the present teachings described herein.

The imaging device 110 is connected to an imaging interface 111 by a cable 112. In embodiments in which the imaging device 110 is a US probe, the cable 112 is a US cable comprising the various electrical signal and power connections required for imaging with a US probe. The imaging interface 111 is illustratively a workstation accessed and operated by a clinician. In certain representative embodiments, the imaging interface 111 is a US workstation adapted to facilitate imaging by the clinician. Again, in a representative embodiment in which the imaging device 110 is a US probe for US imaging, the imaging interface 111 is a US workstation comprising typical components used by a sonographer to carry out the US imaging procedure undertaken. The cable 112 connecting the US probe to the imaging interface generally is a physical connection that extends from the imaging interface 111 to the imaging device 110 and across the physical environment including the floor, an examination table, and other structures between the imaging interface 111 and the imaging device 110. As described more clearly below, various aspects of the present teachings relate to monitoring the location and shape of, and forces acting on the cable 112 during deployment. By application of a trained computational model described in connection with various representative embodiments below, monitoring of the cable 112, including but not limited to analysis of physical forces acting on the cable, forecasting of its remaining usable life before maintenance is required, and the actual three-dimensional (3D) shape of the cable 112 during its deployment. Notably, data from a sensor (not shown in Fig. 1) are provided to the computational model, which when executed by a processor, cause the processor to determine this useful information.

The computer system 115 receives data from the imaging device 110, and stores and processes the data according to the embodiments discussed herein, in addition to the generation of the US imaging data from the raw data received from the imaging device 110. The computer system 115 includes a controller 120, a memory 130, a database 140 and a display 150.

The controller 120 interfaces with the imaging device 110 through an imaging interface 111. The memory 130 stores instructions executable by the controller 120. When executed, and as described more fully below, the instructions cause the controller 120 to implement processes that include monitoring of the cable 112, including, but not limited to analysis of a degree and physical forces acting on the cable, forecasting of it remaining usable life before maintenance is required, and the actual three-dimensional (3D) shape of the cable during its deployment. In addition, the controller 120 may implement additional operations based on executing instructions, such as instructing or otherwise communicating with another element of the computer system 115, including the database 140 and the display 150, to perform one or more of the above-noted processes.

The controller 120 is representative of one or more processing devices and is configured to execute software instructions to perform functions as described in the various embodiments herein. The controller 120 may be implemented by field programmable gate arrays (FPGAs), application specific integrated circuits (ASICs), a general-purpose computer, a central processing unit, a computer processor, a microprocessor, a microcontroller, a state machine, programmable logic device, or combinations thereof, using any combination of hardware, software, firmware, hard-wired logic circuits, or combinations thereof. Additionally, any processing unit or processor herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems, such as in a cloud-based or other multi-site application. The term computing device should also be interpreted to include a collection or network of computing devices each including a processor or processors. Programs have software instructions performed by one or multiple processors that may be within the same computing device or which may be distributed across multiple computing devices.

The memory 130 may include a main memory and/or a static memory, where such memories may communicate with each other and the controller 120 via one or more buses. The memory 130 stores instructions used to implement some, or all aspects of methods and processes described herein. The memory 130 may be implemented by any number, type and combination of random-access memory (RAM) and read-only memory (ROM), for example, and may store various types of information, such as software algorithms, which serves as instructions, which when executed by a processor cause the processor to perform various steps and methods according to the present teachings. For example, in accordance with various representative embodiments, the memory 130 that stores instructions, which when executed by the processor, cause the processor to: to train various computational models according to the representative embodiments. The training of a computational models, which is described more fully below, comprises compiling ground truth data (GTD) from additional, independent and separate sensors able to perform detailed measurements of the cable 112 parameters in addition to and simultaneously with the sensor attached to or disposed over the cable 112; and training a computational model to carry out various functions according to a representative embodiment. These functions include causing the computational model to:
i) determine the type and magnitude of physical forces acting on the cable 112;
ii) forecast the remaining usable life of the cable 112 before maintenance is required;
iii) provide an actual three-dimensional (3D) shape of the cable 112 during its deployment;
iv) predict a position of a clinician handling the imaging device 110;
v) predict movements of the clinician operating the imaging device 110;
vi) determine a position of the imaging device 110 based on the 3D shape of the cable 112 and a 3D position of a cart; and
vii) determine a three-dimensional (3D) surface anatomy based on positions of the imaging device 110.
The computational model, which is an artificial intelligence (AI) model, may be stored in the memory 130. As described more fully below, the computational model may be a known AI model including recurring neural networks (RNNs) and other neural network based models, and computer programs, all of which are executable by the controller 120. The AI model can change in relation to the application and particular embodiment. As such, for the RUL estimation the baseline model may be a LSTM network, whereas for the 3D shape of the cable 112 the baseline model may be a fully connected convolutional network, which includes a detailed physical model (i.e., digital twin) of the cable 112.

More generally, although not necessarily, the compiling of the GTD from measured sensors on or over the cable 112, and the training of various computational model to carry out the above noted functions, may be done using another processor and memory that are not necessarily part of the computer system 115 or the system 100. In this case, after being trained, the computational model may be stored as executable instructions in memory 130, for example, to be executed by a processor of the controller 120. Notably, the computational models may be stored in separate modules in memory. These may be referred to herein as AI modules and include a ML model or DL model trained in accordance with the present teachings such as according to representative embodiments described below. Furthermore, updates to the computational model may also be provided to the computer system 115 and stored in memory 130. Finally, and as will be apparent to one of ordinary skill in the art having the benefit of the present disclosure, according to a representative embodiment, the computational model may be stored in a memory and executed by a processor that are not part of the computer system 115, but rather is connected to the imaging device 110 through an external link (e.g., a known type of internet connection). One advantage of this arrangement is that the sensors, computational model, and outputs can be powered separately (not shown) and thus may operate to sense cable states even when the overlying ultrasound system is powered down. Just by way of illustration, the computational model may be stored as executable instructions in a memory, and executed by a server that is remote from the imaging device (e.g., a CT scanner). When executed by the processor in the remote server, the instructions cause the processor to compile GTD, and train a computational model to carry out various functions according to representative embodiments. These functions include, but are not limited to causing the computational model to:
i) determine the type and magnitude of physical forces acting on the cable 112;
ii) forecast of the remaining usable life of the cable 112 before maintenance is required;
iii) provide the actual three-dimensional (3D) shape of the cable during its deployment; determine an actual three-dimensional (3D) shape of the US cable;
iv) predict a position of a clinician handling the US probe;
v) predict movements of the clinician operating the US probe;
vi) determine a position of the US probe based on a 3D shape of the US cable and a 3D position of a cart; and
vii) determine a three-dimensional (3D) surface anatomy based on positions of the US probe.
The computational model, which is an artificial intelligence (AI) model, may be stored in the memory 130. As described more fully below, the computational model may be a known AI model including recurring neural networks (RNNs) and other neural network based models, and computer programs, all of which are executable by the controller 120.

The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium known in the art. The memory 130 is a tangible storage medium for storing data and executable software instructions and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 130 may store software instructions and/or computer readable code that enable performance of various functions. The memory 130 may be secure and/or encrypted, or unsecure and/or unencrypted.

Similarly, the database 140 stores data and executable instructions used to implement some, or all aspects of methods and processes described herein. As will be described more fully below, the database 140 illustratively stores various data that are used in the training and execution of various computational models of the present teachings. Notably, the database 140 can be foregone, and all data and executable instructions can be stored in memory 130.

The database 140 may be implemented by any number, type and combination of RAM and ROM, for example, and may store various types of information, such as software algorithms, AI models including RNN and other neural network based models, and computer programs, all of which are executable by the controller 120. The various types of ROM and RAM may include any number, type, and combination of computer readable storage media, such as a disk drive, flash memory, EPROM, EEPROM, registers, a hard disk, a removable disk, tape, CD-ROM, DVD, floppy disk, Blu-ray disk, USB drive, or any other form of storage medium known in the art. The database 140 is a tangible storage medium for storing data and executable software instructions that are non-transitory during the time software instructions are stored therein. The database 140 may be secure and/or encrypted, or unsecure and/or unencrypted.

"Memory" and "database" are examples of computer-readable storage media and should be interpreted as possibly being multiple memories or databases. The memory or database may for instance be multiple memories or databases local to the computer, and/or distributed amongst multiple computer systems or computing devices.

The controller 120 illustratively includes or has access to an AI engine, which may be implemented as software that provides artificial intelligence (e.g., inference of a trained network etc.) and applies machine-learning, deep learning etc. described herein. The AI engine, which provides a computational model described below, may reside in any of various components in addition to or other than the controller 120, such as the memory 130, the database 140, an external server, and/or a cloud, for example. When the AI engine is implemented in a cloud, such as at a data center, for example, the AI engine may be connected to the controller 120 via the internet using one or more wired and/or wireless connection(s). The AI engine may be connected to multiple different computers including the controller 120, so that the artificial intelligence and machine-learning described below in connection with various representative embodiments are performed centrally based on and for a relatively large set of medical facilities and corresponding subjects at different locations. Alternatively, the AI engine may implement the artificial intelligence and the machine-learning locally to the controller 120, such as at a single medical facility or in conjunction with a single imaging device 110.

The interface 160 may include a user and/or network interface for providing information and data output by the controller 120 and/or the memory 130 to the user and/or for receiving information and data input by the user. That is, the interface 160 enables the user to enter data and to control or manipulate aspects of the processes described herein, and also enables the controller 120 to indicate the effects of the user's control or manipulation. The interface 160 may include one or more of ports, disk drives, wireless antennas, or other types of receiver circuitry. The interface 160 may further connect one or more user interfaces, such as a mouse, a keyboard, a mouse, a trackball, a joystick, a microphone, a video camera, a touchpad, a touchscreen, voice, or gesture recognition captured by a microphone or video camera, for example.

The display 150 may be a monitor such as a computer monitor, a television, a liquid crystal display (LCD), a light emitting diode (LED) display, a flat panel display, a solid-state display, or a cathode ray tube (CRT) display, or an electronic whiteboard, or a light projector, for example. The display 150 may also provide a graphical user interface (GUI) 155 for displaying and receiving information to and from the user. The display 150 may for example display to a user the findings of a computational model as described herein. For example, a user may be informed of the integrity of a cable, a recommendation to replace the cable based on the RUL, etc.

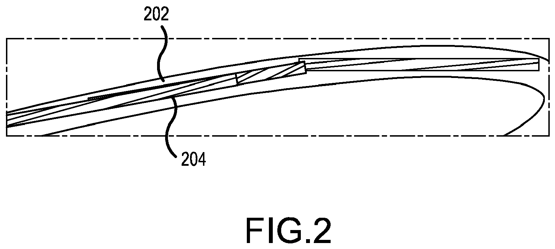

Fig. 2 is a perspective view of a US cable 202 comprising a sensor 204 disposed thereover, according to a representative embodiment. Various aspects and details described above in connection with representative embodiments of Fig. 1 may be common to the representative embodiments described in connection with Fig. 2. These common aspects and details may not be repeated to avoid obscuring the description.

The sensor 204, and any sensor mentioned herein, may be a sensor based on measuring electrical resistance changes of the sensing material. This may include resistive strain gauges and piezoresistive sensors, which modify their electrical resistance based on the amount of strain applied to them. These sensors can be broadly referred to as electrical resistance-based (resistive) strain sensors. The use of these sensors is often preferred over optical fiber-based sensors, the latter of which are typically more expensive. Optical fiber sensors used to measure strain rely on changes in light transmission or reflection within the fiber.

In accordance with a representative embodiment, the sensor 204 comprises a known sensing material commonly referred to as electronic textile ("E-textile") and is disposed along the length of the US cable 202. As shown in Fig. 2, the sensor 204 may comprise a plurality of strips of the E-textile material disposed along the length of the US cable between the workstation (e.g., imaging interface 111) and the US probe (e.g., imaging device 110). Notably, rather than strips of the E-textile material, a continuous section of the E-textile material may be applied to the US cable 202. A plurality of strips of E-textile material or a continuous E-textile material may cover a part of, or the entire cable length. Preferably the sensors cover a substantial part of the total length of the cable. For example, the sensor or plurality of sensors may extend over at least 50%, 60%, 70%, 80%, 90% or 100% of the length of the cable. In certain representative embodiments, the sensor 204 is disposed over a sheath made of the E-textile material that is disposed over the US cable 202. In some embodiments, the sensor 204 is configured to be removeable and/or replaceable from cable 202, by means of temporary adhesives, friction tapes, clips, Velcro^{®} like products, or similar techniques.

E-textile material used in the sensor 204 is a data gathering device and may combine traditional fabrics and fibers with electronics that transmit signals proportional to the force applied to material. E-textiles enable the transfer of data, including sensor data on heat, light, movement, and other local conditions. Electronically integrated textiles are designed primarily with wearable computing in mind, but there are many other applications. As described more fully below, the E-textile material of the sensor 204 is used mainly to monitor properties related to pressure and strain applied to the sensor 204 at specific locations, and thus to the US cable 202 at these same locations. Notably, and just by way of example, data from the sensor 204 at locations where the US cable 202 is known to be subjected to greater and repeated forces than others usefully enable the forecasting of RUL and other important aspects relating to the US cable 202.

Wires embedded in the E-textile material provide voltage signals proportional to the force (e.g., strain or stress) incident on the sensor 204 at a particular moment in time. As such, forces applied to the US cable 202, and thus to the sensor 204 cause a voltage and/or electrical resistance variation across the sensor 204. These voltages/resistances are transmitted along a length of the US cable 202 by a communication link (not shown) and are provided as data related to the forces applied at specific locations of the US cable 202 over time and are stored in memory for use in monitoring the US cable 202 according to representative embodiments described more fully below. In accordance with a representative embodiment, the voltage and/or electrical resistance from the sensor 204 is transmitted as a direct current (DC) analog signal by a comparatively simple electrical connection such as a dedicated electrical cable connected to the sensor 204 along the length of the US cable 202. Sampling of the signal over time by periodic readings (e.g., synchronous or asynchronous time-division multiplexing) by a controller (e.g., controller 120) enables resolution of the particular time and physical position of the sensor 204 where a force is measured. Stated somewhat differently, in accordance with a representative embodiment, voltage and/or electrical resistance signals from the E-textile strip comprising the sensor are conveyed by a dedicated wire running through the E-textile strips. This wire relays, in a sequence at given time intervals (e.g., synchronous or asynchronous time-division multiplexing), analog signals from the E-textile. These intervals may be sampled every couple inches (e.g., every 2" to 4", i.e. every 5 - 10 cm) or at in suitably spaced intervals depending on predetermined knowledge of locations where US cables are more susceptible to damage and relative to the current forces (e.g., strain and pressure) applied to the sensor 204 (and thus the cable 202) at specific locations along the US cable 202. These data are stored and, as described more fully below, are applied to a trained computational model to predict, and forecast certain information related to the deployed US cable 202.

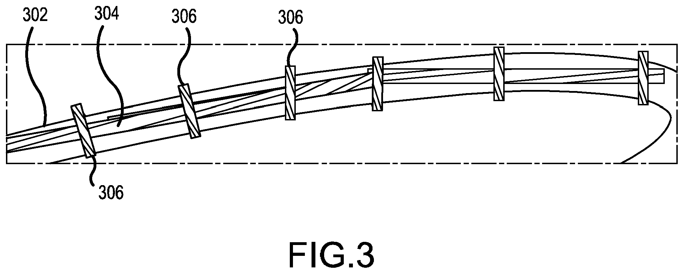

Fig. 3 is a perspective view of a US cable 302 comprising a sensor 304 disposed thereover, according to a representative embodiment. Various aspects and details described above in connection with representative embodiments of Figs. 1 and 2 may be common to the representative embodiments described in connection with Fig. 3. These common aspects and details may not be repeated to avoid obscuring the description.

In accordance with a representative embodiment, the sensor 304 comprises E-textile material disposed along the length of the US cable 302. A plurality of perpendicular sensors 306 are disposed at intervals along the length of the sensor 304. Again, the sensor 304 with the plurality of perpendicular sensors 306 may be in the form of a sheath disposed over the US cable 302. And again, the sheath may be disposed over the US cable in a removable and/or replaceable configuration, or may be integrated over the cable surface itself.

Like the sensor 204, the sensor 304 provides voltage and/or electrical resistance signals proportional to the force (e.g., strain or stress) incident on the sensor 304 at a particular moment in time. As such, forces applied to the US cable 302, and thus to the sensor 304 cause a voltage and/or an electrical resistance across the sensor 304. These voltages and/or electrical resistance are analog values, which are transmitted along a length of the US cable 302 by a communication link (not shown) and are provided as data related to the forces applied at specific locations of the US cable 302 over time (e.g., synchronous or asynchronous time-division multiplexing) and are stored in memory for use in monitoring the US cable 302 according to representative embodiments described more fully below. As such, the sensor 304 functions in very much the same way as the sensor 204 described above.

The perpendicular sensors 306 enable gathering of data indicative of torsion on the US cable 302 at corresponding locations of the perpendicular sensors 306. These data are then stored in memory and entered into a trained computational model to determine the shape of the US cable 302 at a particular moment in time. Notably, and just by way of example, data from the sensor 304 and the perpendicular sensors 306 at locations where the US cable 202 is known to be subjected to greater and repeated forces than at other locations usefully enable the forecasting of RUL and other important aspects relating to the US cable 202.

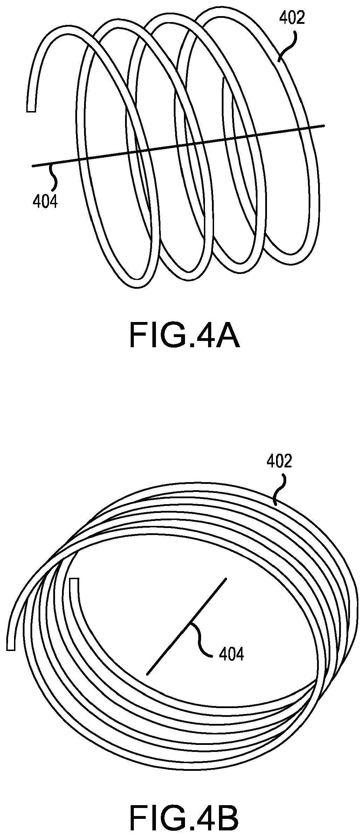

Figs. 4A and 4B are perspective views of a US cable 404 comprising a helicoidal sensor 402 disposed thereover, according to a representative embodiment. Various aspects and details described above in connection with representative embodiments of Figs. 1-3 may be common to the representative embodiments described in connection with Figs. 4A and 4B. These common aspects and details may not be repeated to avoid obscuring the description.

The helicoidal sensor 402 also comprises E-textile material and enables measurements from a curved sensor disposed around the US cable. Notably, sensors 204, 304 provide data that are then used to solve differential equations to determine the shape of the US cable and the forces thereon. By contrast, instead of approximating the curvature of the US cable using perpendicular stripes of E-textile material as described above, the helicoidal sensor 404 enables the direct measure of the curvature and torsion of the US cable 404. Specifically, and as described more fully below, a helicoidal approximation is used to lay the helicoidal sensor 402 on the cable 402 and furthermore to determine the shape and forces acting on the US cable 404. To this end, instead of considering the cable parts between two measuring E-textile points as almost linear, the helicoidal sensor 404 enables the approximation of arcs of a helix. This enables measuring E-textile parts on the cable according to a helicoidal geometry, so to improve the precision of the acquired values of curvature and torsion on the US cable 404. The pressure and stretching values (voltages and/or electrical resistances) from the helicoidal sensor 402 are acquired and provided to a computational model that usefully determines the shape of the US cable 404 as described more fully below. The helicoidal shape of the sensing E-textile element allows a faster and more precise determination of the curve parameters. The reconstructed 3D curve in comparatively more precise and can enable applications that require a very precise (e.g., mm or sub-mm precision) localization of the probe. Notably, and just by way of example, data from the helicoidal sensor 404 at locations where the US cable 404 is known to be subjected to greater and repeated forces than others usefully enable the forecasting of RUL and other important aspects relating to the US cable 404.

The full curve of the helicoidal sensor of the representative embodiments of Figs. 4A and 4B may be represented by the helicoidal mathematical curve representing in detail the shape of the curve: $\left\{\begin{matrix}x \left(θ\right) = ρ cos \left(αθ\right) \\ y \left(θ\right) = ρ sin \left(αθ\right) \\ z \left(θ\right) = θ\end{matrix}\right.$ It is to be noted that the aforementioned helical surface is a minimal surface, i.e., a surface with minimal local area. Moreover, it is to be noted that the helicoidal approximation is valid when the cable is "at rest" (i.e., without any type of solicitation along its whole length, it rests flat) and of uniform section; if the section varies the helicoidal approximation has to be done piecewise along the different cable's sections.

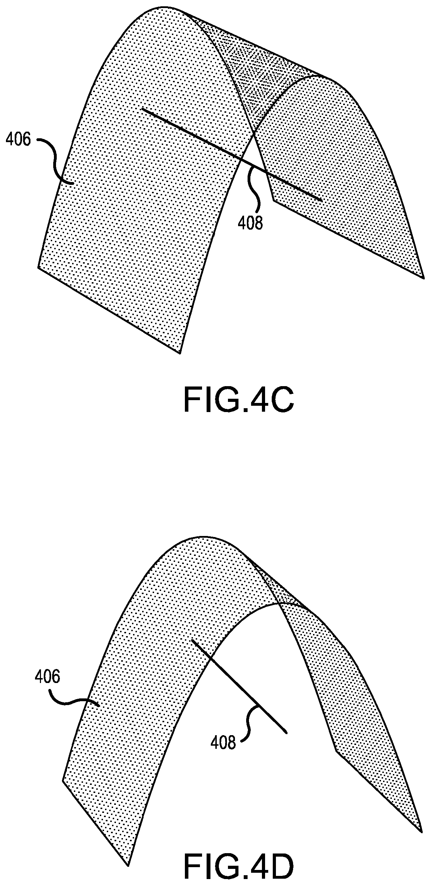

Figs. 4C and 4D are perspective views of a US cable 408 comprising a sensor 406 disposed thereover, according to a representative embodiment. Various aspects and details described above in connection with representative embodiments of Figs. 1-4B may be common to the representative embodiments described in connection with Figs. 4C and 4D. These common aspects and details may not be repeated to avoid obscuring the description.

The representative embodiments shown in Figs. 4C and 4D depict the sensor 406 not being wrapped completely around the US cable 408, but rather shown in another example of what is meant by disposed "over" the US cable 408. As such, like the embodiments described above, the sensor 406 may be considered a "sheath" for the purposes of the present teachings. These are merely illustrative, and are shown to emphasize the various embodiments may be disposed over the US cable 408 with sensors disposed therein. The sensor 406, like previously described sensors, may comprise an E-textile material with sensors therein.

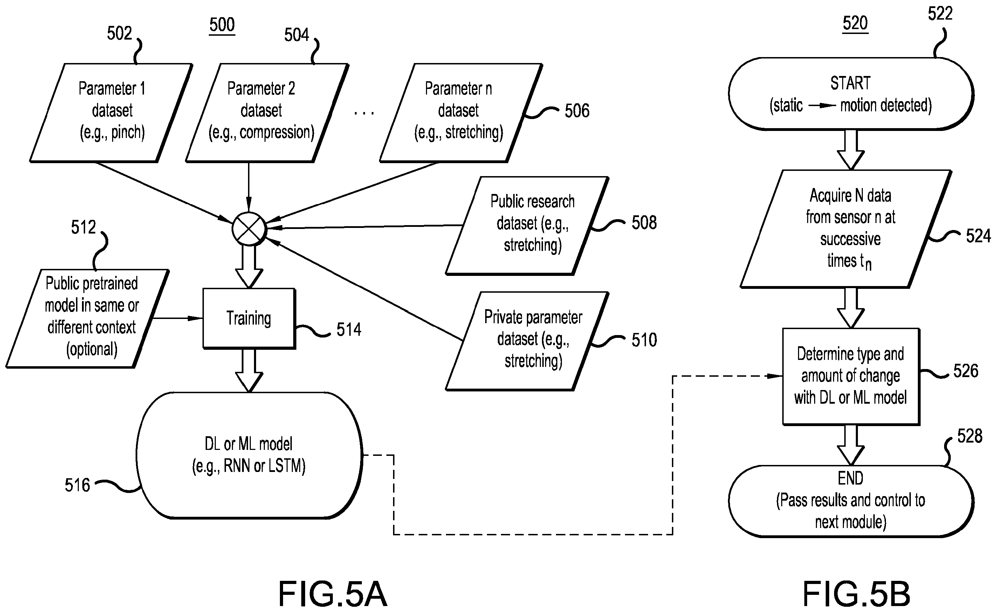

Fig. 5A is a simplified flow diagram showing a method 500 of training a computational model according to a representative embodiment. Various aspects and details described above in connection with representative embodiments of Figs. 1-4D may be common to the representative embodiments described in connection with Fig. 5A. These common aspects and details may not be repeated to avoid obscuring the description. The method of training the computational model described in connection with Fig. 5A is contemplated for not only the method, but also the tangible, non-transitory computer-readable medium that stores instructions, which when executed by a processor cause the processor to carry out the method of training the computational model.

As shown, several parameters are provided to train the computational model in accordance with a representative embodiment. Since the currently described computational model is directed to determining a type and an amount of physical change in a US cable, parameters related to these determinations are made. These parameters may be measured by sensors, such as sensors 204, 304, 404 as described above, although other types of sensors and other parameters useful in determining a type and an amount of physical change in the US cable are contemplated. Notably, data for n (n=positive integer) parameters are gathered by j (j=positive integer) sensors disposed along the length of the US cable. In accordance with representative embodiments, these n parameters from j sensors are gathered in sufficient number to beneficially improve the accuracy of the trained computational model.

In accordance with the presently described representative embodiment, a first parameter data set 502 comprises data related to a pinch of the US cable as may occur when the US cable is moved and manipulated during a US examination. These pinches of the US cable are manifest as forces, which when applied to the sensors on the US cable, cause a voltage and/or electrical resistance change in the sensor. The voltages and/or electrical resistance values are provided by the sensor to a memory, and ultimately provided for training the computational model at 514. Notably, these pinch data may be compiled together with some other reference sensors' data as GTD and are used in the training of the computational model. In accordance with a representative embodiment, the GTD gathered for training the computational model comprises a significant amount of data and may be gathered in a variety of settings to provide an ample set of GTD for an acceptable degree of training. Furthermore, and as alluded to above, the first parameter data set 502 may comprise not only data gathered at various locations of the US cable, but also at locations known to cause greater forces than others. Just by way of illustration, for example, at locations where the US cable is attached to the workstation (e.g., imaging interface 111) or the US probe (e.g., imaging device 110) are known to be so-called 'trouble-spots' where the wear and tear on the US cable tend to be more extreme than other locations on the US cable. Gathering GTD related to the first parameter data 502 set can thus be useful, for example, in monitoring the health of the US cable at points of attachment.

In accordance with the presently described representative embodiment, a second parameter data set 504 comprises data related to a compression of the US cable as may occur during a US examination. These compressions of the US cable are manifest as forces, which when applied to the sensors on the US cable cause a voltage and/or electrical resistance change in the sensor. The voltages and/or electrical resistance values are provided by the sensor to a memory, and ultimately provided for training the computational model at 514. Notably, these compression data may be compiled together with some other reference sensors' data as GTD and are used in the training of the computational model. In accordance with a representative embodiment, the GTD gathered for training the computational model comprises a significant amount of data and may be gathered in a variety of settings to provide an ample set of GTD for an acceptable degree of training. Furthermore, and as alluded to above, the second parameter data set 504 may comprise not only data gathered at various locations of the US cable, but also at locations known to cause greater forces than others. Just by way of illustration, for example, at locations near the feet of the sonographer, examination table, or workstation, the US cable are known to be so-called 'trouble-spots' where the cable can be stepped on or ridden over, thereby causing wear and tear on the US cable that tends to be more extreme than other locations on the US cable. Gathering GTD related to the second parameter data set 504 can thus be useful, for example, in monitoring the health of the US cable at locations susceptible to such compressions.

In accordance with the presently described representative embodiment, an nth parameter data set 506 comprises data related to stretching of the US cable as may occur during a US examination. The stretching of the US cable is manifest as forces, which when applied to the sensors on the US cable, cause a voltage and/or electrical resistance change in the sensor. The voltages are provided by the sensor to a memory, and ultimately provided for training the computational model at 514. Notably, the data on stretching of the US cable may be compiled together with some other reference sensors' data as GTD and are used in the training of the computational model. In accordance with a representative embodiment, the GTD gathered for training the computational model comprises a significant amount of data and may be gathered in a variety of settings to provide an ample set of GTD for an acceptable degree of training. Furthermore, and as alluded to above, the nth parameter data set 506 may comprise not only data gathered at various locations of the US cable, but also at locations known to cause greater forces than others. Again, data related to stretching at so-called 'trouble-spots' where the greater stress is applied to the US may cause causing wear and tear on the US cable that tend to be more extreme than other locations on the US cable. Gathering GTD related to the nth parameter data set 506 can thus be useful, for example, in monitoring the health of the US cable at locations susceptible to such compressions.

Other parameters are provided for training the computational model at 514. For example, publicly available parameter data set 508 may be used for training the model. These data may have been gathered from a significant number of uses of US cables in a variety of other machines. Generally, the publicly available parameter data set 508 comprises germane data useful in developing a computational model to monitor a US cable in a number of ways, such as noted above. As such, the data from the publicly available parameter data set 508 include one or more of the n-parameters of datasets 502~506 noted above. The publicly available parameter data set 508 may comprise a great volume of data which, as alluded to above, can improve the accuracy of the computational model. Moreover, by applying these data to the training at 514, the speed with which a viable computational model is trained can be improved, thereby improving the efficiency of the training of the computational model.

In addition to publicly available parameter data set 508, a privately available parameter data set 510 may also be provided to train the model at 514. These data may be gathered during calibration and helped to shape and define the ground truth procedure for the acquisition of the cable parameters. These data are extremely useful since they are acquired in a controlled and uniform environment and are therefore highly reliable. The privately available parameter data set 510 comprises germane data useful in developing a computational model to monitor a US cable in a number of ways, such as noted above. As such, the data from the privately available parameter data set 510 include one or more of the n-parameters of datasets 502~506 noted above. The privately available data parameter set 510 may comprise a great volume of data which, as alluded to above, can improve the accuracy of the computational model. Moreover, by applying these data to the training 514, the speed with which a viable computational model is trained can be improved, thereby improving the efficiency of the training of the computational model.

Another way to improve the efficiency of the training at 514 is by the use of a previously trained computational model 512. This previously trained computational model 512 is illustratively from a same context as the model being trained at 514. However, this is not essential as trained models from other contexts including for example, image recognition, forecasting and the like. These models are, in general, but not exclusively, recognized as foundational models that may be provided to the training at 514. Notably, the use of a previously trained model in the training at 514 allows the training sequence to be limited only to the last layers of the computational model, and as such increases substantially the speed with which the training at 514 is completed.

The GTD from the various sources are then compiled from the sensor data from a plurality of locations of the US sensor, including each of a plurality of specific locations where wear and tear are known to be greater, and the training of the computational model to monitor the US cable and make certain inferences regarding the US cable using the GTD.

At 514, training of the computational model (or AI model, or machine-learning (ML) algorithm) is carried out for the first pass. Training of the computational model generally happens during product development, and as noted above may not be done using computer system 115, but more generally may be completed using another memory and processor, with the trained computational model being stored in memory 130. The training of the computational model is based on ground-truth measurements of the n-parameters from a plurality of locations of the US sensor, including each of a plurality of specific locations where wear and tear are known to be greater.

The training sequence of the computational model is carried out using one of a number of known machine-learning techniques known to those of ordinary skill in the art of AI and mathematical models. In machine-learning, an algorithmic model "learns" how to transform its input data into meaningful output. During the learning sequence, the computational model is shown known examples of inputs and their correct or desired output. These examples are called ground truth, and in accordance with the present teachings comprise the GTD gathered at 502~510. During the learning sequence, the computational model adjusts its inner parameters given the input parameters of the examples and produces the corresponding meaningful output or so-called target. The adjustment process is guided by instructions on how to measure the distance between the currently produced output and the desired output and applying some form of gradient adjustment to the learned parameters of the model. These instructions are called the objective function.

In deep learning (DL), which is a subfield of machine-learning, the inner parameters inside the computational model are organized into successively connected layers, where each layer produces, typically, increasingly meaningful output as input to the next layer, until the last layer which produces the final output. Deep learning layers are typically implemented as so-called neural networks, that is, layers are comprised of a set of nodes, each node representing an output value and a prescription on how to compute this output value from a subset of output values of the previous layer's nodes. The prescription being a weighted sum of transformed output values of the previous layer's nodes, each node only needs to store the weights to reproduce the node's activation value from the same of the previous layer going back to the input. The transformation function is the same for all nodes in a layer and is also called activation function. There are a limited number of activation functions that are used today. A particular way to set which previous layer's nodes provide input to a next layer's node is convolution. Networks based on this way are called convolutional networks.

Thus, in the learning phase or so-called training, for each example, the output of the final layer is computed. Outputs for all examples are compared with the desired outputs by way of the objective function. The output of the objective function, the so-called loss, is used as a feedback signal to adjust the weights such that the loss is reduced. The adjustment, i.e., which weights to change and by how much, is computed by the central algorithm of deep learning, so-called backpropagation, which is based on the fact that the weighted sums that connect the layer nodes are functions that have simple derivatives. The adjustment is iterated until the loss reaches a prescribed threshold or no longer changes significantly.

A deep-learning network thus can be stored (e.g., in database 140 or memory 130) as a topology that describes the layers and activation functions and a (large) set of weights (simply values). A trained network is the same, only the weights are now fixed to particular values. Once the network is trained, it is put to use, that is, to predict output for new input for which the desired output is unknown.

In accordance the present teachings, the computational model is stored as instructions to provide the machine-learning algorithm, such as a convolutional neural-network deep learning algorithm. When executed by a processor, the machine-learning algorithm (the computational model) of the representative embodiments is used to predict cable integrity of the US cable based on ground truth data, or to forecast a remaining useful life (RUL) of the US cable using ground truth data, or to determine an actual shape of the US cable, or any combination thereof.

Finally, after completion of the training of the computational model at 514, the trained computational model is output at 516. The trained computational model, which may be a recurrent neural network (RNN) or a Long Short-Term Memory (LSTM) network, is then stored in memory for execution according to the method of Fig. 5B.

Fig. 5B is a simplified flow diagram showing a method 520 of inferring a type and a magnitude of a physical change to a US cable using the computational model trained according to the method 500 in accordance with a representative embodiment. Various aspects and details described above in connection with representative embodiments of Figs. 1-5A may be common to the representative embodiments described in connection with Fig. 5B. These common aspects and details may not be repeated to avoid obscuring the description. Most notably, the method 520 is contemplated for not only the method, but also for storing in a tangible, non-transitory computer-readable medium that stores instructions, which when executed by a processor, cause the processor to carry out the method of inferring (predicting) a type and a magnitude of a physical change to a US cable.

The method starts at 522 where motion of the US cable is detected, or forces incident on the US cable, or both are sensed. The motion may be detected via a motion sensor, an accelerometer, a vibration sensor or when forces incident on the cable are sensed through any of the sensors 204, 304, 306 and 404. Once motion is detected a motion event starts, wherein the cable is being moved. The motion event is terminated or finishes when no motion is detected anymore.

At 524, N data samples are acquired from sensors disposed over the US cable. These N data samples include data of the n-parameters of 502~506, and other parameters, such as publicly and privately available parameter data sets 508, 510. These data are at successive times tₙ from a US machine comprising the US probe and the US cable under, where the different time slots tₙ₋₁, tₙ represent a synchronous or asynchronous time-division multiplexing able to use a single signaling wire to transmit the data of the N sensors available. Notably, the signaling wire may be accompanied also by the necessary additional wires and/or mechanisms to effect the error correction and/or synchronization and/or sensor's data availability in the asynchronous case. The involved signals can be both of the analog and the digital nature.

At 526, the N data samples are provided to the trained computational model to determine the type and amount of change to the US cable. Notably, the computational model applied at 526 may be a trained ML or a trained DL model such as described above.

As alluded to above, the data related to forces (e.g., compression and stretching) are used to determine (using an illustrative AI module - ML or DL - stored as executable instructions in memory) the presence of some stress on the cable, the level of such stress, the corresponding location/s on the cable and the type of stress (pinching, compressing etc.). In addition, and as described more fully below, the execution of the AI model beneficially updates forecasting of the cable RUL, and the probability distribution of the RUL along the cable length. The resultant RUL is stored and used to communicate to the user if any action is needed and to show the current cable reliability level, and eventual stressing points. Just by way of example, a stressing event caused by a US cable going under a cart wheel or the like may cause an alert signal to be issued warning that the US cable is susceptible to pinching and damage simply by knowledge of the location.

At 528, the method ends with the output of results to a next module, illustratively the controller/state machine that governs the whole device process. Again, and just by way of illustration, the output from 528 may include information on the location and magnitude of a number of forces applied to the US cable, including, but not limited to, locations where US cables are prone to breakdown or breakage.

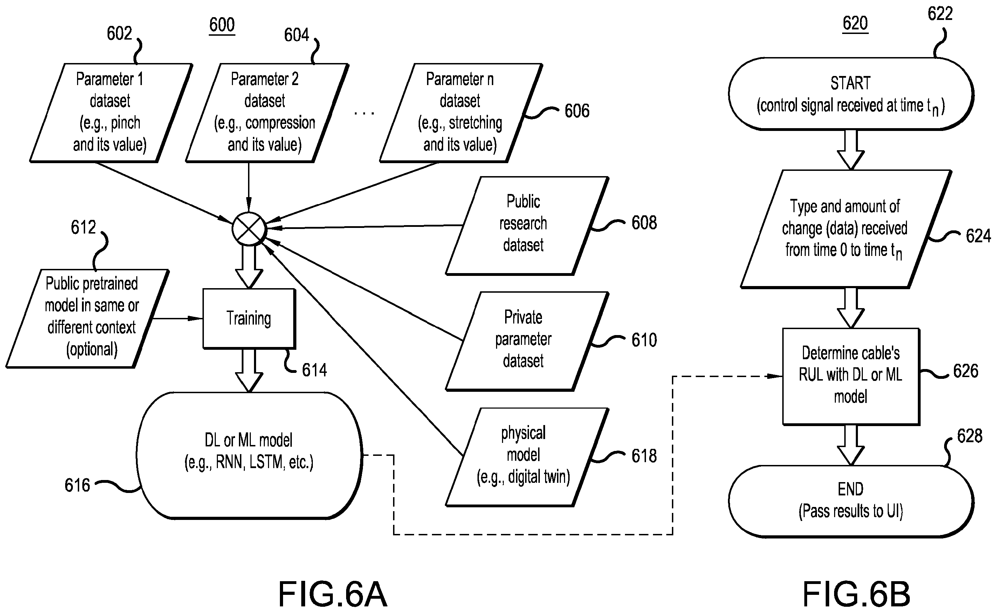

Fig. 6A is a simplified flow diagram showing a method of training a computational model to predict the RUL of a US cable according to a representative embodiment. Various aspects and details described above in connection with representative embodiments of Figs. 1-5B may be common to the representative embodiments described in connection with Fig. 6A. These common aspects and details may not be repeated to avoid obscuring the description. The method of training the computational model described in connection with Fig. 6A is contemplated for not only the method, but also the tangible, non-transitory computer-readable medium that stores instructions, which when executed by a processor cause the processor to carry out the method of training the computational model.

As shown, a number of parameters are provided to train the computational model in accordance with a representative embodiment. Since the currently described computational model is directed to determining an RUL in a US cable, parameters related to these determinations are made. These parameters are contemplated to be measured by sensors, such as sensors 204, 304, 404 as described above, although other sensors and other parameters useful in determining a type and an amount of physical change in the US cable are contemplated. Notably, data for n (n=positive integer) parameters are gathered by j (j=positive integer) sensors disposed along the length of the US cable. In accordance with representative embodiments, these n parameters from j sensors are gathered in sufficient number to beneficially improve the accuracy of the trained computational model.

In accordance with the presently described representative embodiment, a first parameter data set 602 comprises data related to a pinch of the US cable as may occur when the US cable is moved and manipulated during a US examination. These pinches of the US cable are manifest as forces, which when applied to the sensors on the US cable cause a voltage and/or electrical resistance change in the sensor. The voltages and/or electrical resistance values are provided by the sensor to a memory, and ultimately provided for training the computational model at 614. Notably, these pinch data may be compiled together with some other reference sensors' data as ground truth data (GTD) and are used in the training of the computational model. In accordance with a representative embodiment, the GTD gathered for training the computational model comprises a significant amount of data and may be gathered in a variety of settings to provide an ample set of GTD for an acceptable degree of training. Furthermore, and as alluded to above, the first parameter data set 602 may comprise not only data gathered at various locations of the US cable, but also at locations known to cause greater forces than others. Just by way of illustration, for example, at locations where the US cable is attached to the workstation (e.g., imaging interface 111) or the US probe (e.g., imaging device 110) are known to be so-called 'trouble-spots' where the wear and tear on the US cable tends to be more extreme than other locations on the US cable. Gathering GTD related to the first parameter data 602 set can thus be useful, for example, in monitoring the health of the US cable at points of attachment.

In accordance with the presently described representative embodiment, a second parameter data set 604 comprises data related to a compression of the US cable as may occur during a US examination. These compressions of the US cable are manifest as forces, which when applied to the sensors on the US cable cause a voltage and/or electrical resistance change in the sensor. The voltages and/or electrical resistance values are provided by the sensor to a memory, and ultimately provided for training the computational model at 614. Notably, these compression data may be compiled together with some other reference sensors' data as GTD and are used in the training of the computational model. In accordance with a representative embodiment, the GTD gathered for training the computational model comprises a significant amount of data and may be gathered in a variety of settings to provide an ample set of GTD for an acceptable degree of training. Furthermore, and as alluded to above, the second parameter data set 604 may comprise not only data gathered at various locations of the US cable, but also at locations known to cause greater forces than others. Just by way of illustration, for example, at locations near the feet of the sonographer, examination table, or workstation, the US cable are known to be so-called 'trouble-spots' where the cable can be stepped on or ridden over, thereby causing wear and tear on the US cable that tends to be more extreme than other locations on the US cable. Gathering GTD related to the second parameter data set 604 can thus be useful, for example, in monitoring the health of the US cable at locations susceptible to such compressions.

In accordance with the presently described representative embodiment, an nth parameter data set 606 comprises data related to stretching of the US cable as may occur during a US examination. The stretching of the US cable is manifest as forces, which when applied to the sensors on the US cable cause a voltage and/or electrical resistance change in the sensor. The voltages and/or electrical resistance values are provided by the sensor to a memory, and ultimately provided for training the computational model at 614. Notably, the data on stretching of the US cable may be compiled together with some other reference sensors' data as GTD and are used in the training of the computational model. In accordance with a representative embodiment, the GTD gathered for training the computational model comprises a significant amount of data and may be gathered in a variety of settings to provide an ample set of GTD for an acceptable degree of training. Furthermore, and as alluded to above, the nth parameter data set 606 may comprise not only data gathered at various locations of the US cable, but also at locations known to cause greater forces than others. Again, data related to stretching at so-called 'trouble-spots' where the greater stress is applied to the US may cause causing wear and tear on the US cable that tends to be more extreme than other locations on the US cable. Gathering GTD related to the nth parameter data set 606 can thus be useful, for example, in monitoring the health of the US cable at locations susceptible to such compressions.

Other parameters are provided for training the computational model at 614 to forecast the RUL of a US cable. For example, publicly available parameter data set 608 may be used for training the model. These data may have been gathered from a significant number of uses of US cables in a variety of other machines. Generally, the publicly available parameter data set 608 comprises germane data useful in developing a computational model to monitor a US cable in a number of ways, such as noted above. As such, the data from the publicly available parameter data set 608 include one or more of the n-parameters of parameter datasets 602~606 noted above. The publicly available parameter data set 608 may comprise a great volume of data which, as alluded to above, can improve the accuracy of the computational model. Moreover, by applying these data to the training 614, the speed with which a viable computational model is trained can be improved, thereby improving the efficiency of the training of the computational model.

In addition to publicly available parameter data set 608, a privately available parameter data set 610 may also be provided to train the model at 614. These data may be gathered, for example, in a calibration sequence or similar experiment during development of the computational models implemented in connection with various representative embodiments of the present teachings. These data have helped to shape and define the ground truth procedure for the acquisition of the cable parameters, and are useful because they are comparatively highly reliable having been acquired in a controlled and uniform environment. The privately available parameter data set 610 comprises germane data useful in developing a computational model to monitor a US cable in a number of ways, such as noted above. As such, the data from the publicly available parameter data set 608 include one or more of the n-parameters of datasets 602~606 noted above. The privately available parameter data set 610 may comprise a great volume of data which, as alluded to above, can improve the accuracy of the computational model. Moreover, by applying these data to the training 614, the speed with which a viable computational model is trained can be improved, thereby improving the efficiency of the training of the computational model.

Another way to improve the efficiency of the training at 614 is by the use of a previously trained computational model 612. This previously trained computational model 612 is illustratively from a same context as the model being trained at 614. However, this is not essential as trained models from other contexts including for example, but not limited to, image recognition, forecasting and the like. These models are, in general, but not exclusively, recognized as foundational models and may be provided to the training may at 614. Notably, the use of a previously trained model in the training at 614 allows the training sequence to be limited only to the last layers of the computational model, and as such increases the speed with which the training at 614 is completed.

A digital twin at 618 is built by a mathematical model (e.g., Finite Element Model - FEM etc.), and is a type of simulation in which the data are updated frequently from the sensors deployed along the US cable. To this end, this digital model includes not only the main geometric features of the cable (e.g., length, thickness, etc.), but also its composition in term of constituent materials (e.g., external skin or sheath, copper cabling, shielding etc.), its electrical model (e.g., electromagnetic behavior), its mechanical, thermal and components materials' model, its combustion characteristics, etc. This model has been complemented, through an iterative optimization technique, by additional data derived from an artificial intelligence, machine learning and other mathematical methods based on the data of similarly designed cables; this process leads to improved accuracy of the digital twin model. In particular, we measured in the lab and used in the digital twin for the electrical part the electrical values (e.g., electrical resistance etc.) of the conductor material and its type (copper or aluminum), the cross-sectional area of the conductors, the type of conductors (solid or stranded) and the polymer components used (insulation, bedding and sheath); for the mechanical part the measurements focused primarily on elastic properties - stretching stiffness, bending stiffness, cable weight, length etc. A further set of measured values used in the digital twin model refer to the mechanical values associated with the contact, friction, collision, and fluid-structure interactions.

At 618 a physical model of the US cable is provided as another parameter for training at 614. The physical model is a simulation of the entire US cable, whereas other parameters provided for training at 614 relate to a portion or aspect of the US cable. This physical model may be a digital twin and is a simulation of the entire US cable at different moments in time with the added value of real sensors' data added. As will be appreciated the digital twin represents physical reality of the US cable and may be useful in assessment of the cable in a variety of ways, such as forecasting the RUL.

The GTD together with some other reference sensors' data from the various sources are then compiled from a plurality of locations of the US sensor, including each of a plurality of specific locations where wear and tear are known to be greater, and the training of the computational model to monitor the US cable and make certain inferences regarding the US cable using the GTD.

At 614, training of the computational model (or AI model, or machine-learning (ML) algorithm) is carried out for the first pass. Training of the computational model generally happens during product development, and as noted above may not be done using computer system 115, but more generally may be completed using another memory and processor, with the trained computational model being stored in memory 130. The training of the computational model is based on ground-truth measurements of the n-parameters from a plurality of locations of the US sensor, including each of a plurality of specific locations where wear and tear are known to be greater.

The training sequence of the computational model is carried out using one of a number of known machine-learning techniques known to those of ordinary skill in the art of AI and mathematical models. In machine-learning, an algorithmic model "learns" how to transform its input data into meaningful output. During the learning sequence, the computational model is shown known examples of inputs and their correct or desired output. These examples are called ground truth, and in accordance with the present teachings comprise the GTD gathered at 602~610 and 618. During the learning sequence, the computational model adjusts its inner parameters given the input parameters of the examples and produces the corresponding meaningful output or so-called target. The adjustment process is guided by instructions on how to measure the distance between the currently produced output and the desired output. These instructions are called the objective function. At 616, the trained and tested DL/ML computational model is provided.

Fig. 6B is a simplified flow diagram showing a method 620 of inferring a type and a magnitude of a physical change to a US cable using the computational model trained according to the method 620 in accordance with a representative embodiment. Various aspects and details described above in connection with representative embodiments of Figs. 1-6A may be common to the representative embodiments described in connection with Fig. 6B. These common aspects and details may not be repeated to avoid obscuring the description. Most notably, the method 620 is contemplated for not only the method, but also for storing in a tangible, non-transitory computer-readable medium that stores instructions, which when executed by a processor, cause the processor to carry out the method of inferring (predicting) an RUL of a US cable.

The method starts at 622 where motion of the US cable is detected, or forces incident on the US cable, or both, are sensed. The motion may be detected via a motion sensor, an accelerometer, a vibration sensor or when forces incident on the cable are sensed through any of the sensors 204, 304, 306 and 404.

At 624, N data samples are acquired from sensors disposed over the US cable. These N data samples include data of the n-parameters of 602~606, and other parameters such as publicly and privately available parameter data sets 608, 610. These data are at successive times tₙ from a US machine comprising the US probe and the US cable making the various connections for proper function.

At 626, the N data samples are provided to the trained computational model to determine the type and amount of change to the US cable. Notably, the computational model applied at 626 may be a trained ML or a trained DL model such as described above.

At 628, the method ends with the output of results to a next user interface module for distribution and visualization and stored for further use. Again, and just by way of illustration, the output from 628 may include information on the RUL of the US cable. Such information may for example be provided directly as a message to the system display 150, may be stored in memory 130 for subsequent retrieval by a service technician, or may be transmitted by wired or wireless known means, such as WiFi or internet transmission, to a remote service provider.

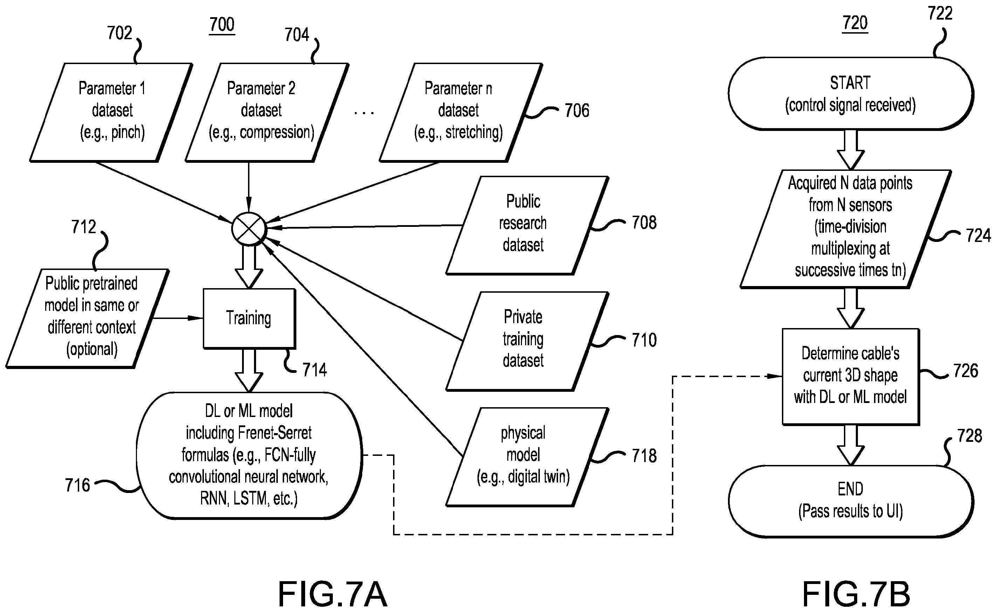

Fig. 7A is a simplified flow diagram showing a method of training a computational model to predict the actual shape of a US cable according to a representative embodiment. Various aspects and details described above in connection with representative embodiments of Figs. 1-6B may be common to the representative embodiments described in connection with Fig. 7A. These common aspects and details may not be repeated to avoid obscuring the description. The method of training the computational model described in connection with Fig. 7A is contemplated for not only the method, but also the tangible, non-transitory computer-readable medium that stores instructions, which when executed by a processor cause the processor to carry out the method of training the computational model.

As shown, a number of parameters are provided to train the computational model in accordance with a representative embodiment. Since the currently described computational model is directed to determining an actual shape of the US cable, parameters related to these determinations are made. These parameters are contemplated to be measured by sensors, such as sensors 204, 304, 404 as described above, although other sensors and other parameters useful in determining a type and an amount of physical change in the US cable are contemplated. Notably, data for n (n=positive integer) parameters are gathered by j (j=positive integer) sensors disposed along the length of the US cable. In accordance with representative embodiments, these n parameters from j sensors are gathered in sufficient number to beneficially improve the accuracy of the trained computational model.

In accordance with the presently described representative embodiment, a first parameter data set 702 comprises data related to a pinch of the US cable as may occur when the US cable is moved and manipulated during a US examination. These pinches of the US cable are manifest as forces, which when applied to the sensors on the US cable cause a voltage and/or electrical resistance change in the sensor. The voltages and/or electrical resistance values are provided by the sensor to a memory, and ultimately provided for training the computational model at 714. Notably, these pinch data may be compiled together with some other reference sensors' data as GTD and are used in the training of the computational model. In accordance with a representative embodiment, the GTD gathered for training the computational model comprises a significant amount of data and may be gathered in a variety of settings to provide an ample set of GTD for an acceptable degree of training. Furthermore, and as alluded to above, the first parameter data set 702 may comprise not only data gathered at various locations of the US cable, but also at locations known to cause greater forces than others. Just by way of illustration, for example, at locations where the US cable is attached to the workstation (e.g., imaging interface 111) or the US probe (e.g., imaging device 110) are known to be so-called 'trouble-spots' where the wear and tear on the US cable tends to be more extreme than other locations on the US cable. Gathering GTD related to the first parameter data set 702 set can thus be useful, for example, in monitoring the health of the US cable at points of attachment.

In accordance with the presently described representative embodiment, a second parameter data set 704 comprises data related to a compression of the US cable as may occur during a US examination. These compressions of the US cable are manifest as forces, which when applied to the sensors on the US cable cause a voltage and/or electrical resistance change in the sensor. The voltages and/or electrical resistance values are provided by the sensor to a memory, and ultimately provided for training the computational model at 714. Notably, these compression data may be compiled together with some other reference sensors' data as GTD and are used in the training of the computational model. In accordance with a representative embodiment, the GTD gathered for training the computational model comprises a significant amount of data and may be gathered in a variety of settings to provide an ample set of GTD for an acceptable degree of training. Furthermore, and as alluded to above, the second parameter data set 704 may comprise not only data gathered at various locations of the US cable, but also at locations known to cause greater forces than others. Just by way of illustration, for example, at locations near the feet of the sonographer, examination table, or workstation, the US cable are known to be so-called 'trouble-spots' where the cable can be stepped on or ridden over, thereby causing wear and tear on the US cable that tends to be more extreme than other locations on the US cable. Gathering GTD related to the second parameter data set 704 can thus be useful, for example, in monitoring the health of the US cable at locations susceptible to such compressions.

In accordance with the presently described representative embodiment, an nth parameter data set 706 comprises data related to stretching of the US cable as may occur during a US examination. The stretching of the US cable is manifest as forces, which when applied to the sensors on the US cable cause a voltage and/or electrical resistance change in the sensor. The voltages and/or electrical resistance values are provided by the sensor to a memory, and ultimately provided for training the computational model at 714. Notably, the data on stretching of the US may be compiled as GTD and are used in the training of the computational model. In accordance with a representative embodiment, the GTD gathered for training the computational model comprises a significant amount of data and may be gathered in a variety of settings to provide an ample set of GTD for an acceptable degree of training. Furthermore, and as alluded to above, the nth parameter data set 706 may comprise not only data gathered at various locations of the US cable, but also at locations known to cause greater forces than others. Again, data related to stretching at so-called 'trouble-spots' where the greater stress is applied to the US may cause causing wear and tear on the US cable that tends to be more extreme than other locations on the US cable. Gathering GTD related to the nth parameter data set 706 can thus be useful, for example, in monitoring the health of the US cable at locations susceptible to such compressions.

Other parameters are provided for training the computational model at 714 to estimate the shape of a US cable. For example, publicly available parameter data set 708 may be used for training the model. These data may have been gathered from a significant number of uses of US cables in a variety of other machines. Generally, the publicly available parameter data set 708 comprises germane data useful in developing a computational model to monitor a US cable in a number of ways, such as noted above. As such, the data from the publicly available parameter data set 708 include one or more of the n-parameters of parameter datasets 702~706 noted above. The publicly available parameter data set 708 may comprise a great volume of data which, as alluded to above, can improve the accuracy of the computational model. Moreover, by applying these data to the training 714, the speed with which a viable computational model is trained can be improved, thereby improving the efficiency of the training of the computational model.

In addition to publicly available parameter data set 708, a privately available parameter data set 710 may also be provided to train the model at 714. As described above these data may be gathered, for example, in a calibration sequence or similar experiment during development of the computational models implemented in connection with various representative embodiments of the present teachings. These data have helped to shape and define the ground truth procedure for the acquisition of the cable parameters, and are useful because they are comparatively highly reliable having been acquired in a controlled and uniform environment. The privately available parameter data set 710 comprises germane data useful in developing a computational model to monitor a US cable in a number of ways, such as noted above. As such, the data from the privately available parameter data set 710 include one or more of the n-parameters of parameter datasets 702~706 noted above. The privately available parameter data set 710 may comprise a great volume of data which, as alluded to above, can improve the accuracy of the computational model. Moreover, by applying these data to the training 714, the speed with which a viable computational model is trained can be improved, thereby improving the efficiency of the training of the computational model.

Another way to improve the efficiency of the training at 714 is by the use of a previously trained computational model 712. This trained computational model 712 is illustratively from a same context as the model being trained at 714. However, this is not essential as trained models from other contexts may be provided to the training at 714. These trained models from other contexts include for example, but not limited to, image recognition, forecasting and the like. These models are, in general, but not exclusively, recognized as foundational models and may be provided to the training. Notably, the use of a previously trained computational model in the training at 714 allows the training sequence to be limited only to the last layers of the computational model, and as such increases the speed with which the training at 714 is completed.

A digital twin model at 718 is built by a mathematical model such as by a Finite Element Model (FEM), and is a type of simulation in which the data are updated frequently from the sensors deployed along the US cable. To this end, this digital model includes not only the main geometric features of the cable (e.g., length, thickness. etc.), but also its composition in term of constituents materials (e.g., external skin or sheath, copper cabling, shielding etc.), its electrical model (e.g., electromagnetic behavior), its mechanical, thermal and components materials' model, its combustion characteristics, etc. This model has been complemented, through an iterative optimization technique, by additional data derived from an artificial intelligence, machine learning and other mathematical methods based on the data of similarly designed cables; this process leads to improved accuracy of the digital twin model. In particular, we measured in the lab and used in the digital twin for the electrical part the electrical values (e.g., electrical resistance etc.) of the conductor material and its type (copper or aluminum), the cross-sectional area of the conductors, the type of conductors (solid or stranded) and the polymer components used (insulation, bedding and sheath); for the mechanical part the measurements focused primarily on elastic properties - stretching stiffness, bending stiffness, cable weight, length etc. A further set of measured values used in the digital twin model refer to the mechanical values associated with the contact, friction, collision, and fluid-structure interactions.

At 718 a physical model of the US cable is provided as another parameter for training at 714. The physical model is a simulation of the entire US cable, whereas other parameters provided for training at 714 relate to a portion or aspect of the US cable. This physical model may be referred to as a digital twin and is a simulation of the entire US cable at different moments in time. As will be appreciated the digital twin represents physical reality of the US cable and may be useful in assessment of the cable in a variety of ways, such as estimating the cable shape, e.g. its arcuate physical position and shape in 3 dimensional space.

The GTD from the various sources together with some other reference sensors' data are then compiled from the sensor data from a plurality of locations of the US sensor, including each of a plurality of specific locations where wear and tear are known to be greater, and the training of the computational model to monitor the US cable and make certain inferences regarding the US cable using the GTD.

At 714, training of the computational model (or AI model, or machine-learning - ML - algorithm) is carried out for the first pass. Training of the computational model generally happens during product development, and as noted above may not be done using computer system 115, but more generally may be completed using another memory and processor, with the trained computational model being stored in memory 130. The training of the computational model is based on ground-truth measurements of the n-parameters from the plurality of locations of the US sensor, including each of a plurality of specific locations where wear and tear are known to be greater.

The training sequence of the computational model is carried out using one of a number of known machine-learning techniques known to those of ordinary skill in the art of AI and mathematical models. In machine-learning, an algorithmic model "learns" how to transform its input data into meaningful output. During the learning sequence, the computational model is shown known examples of inputs and their correct or desired output. These examples are called ground truth, and in accordance with the present teachings comprise the GTD gathered at 702~710 and 718 together with some other reference sensors' data. During the learning sequence, the computational model adjusts its inner parameters given the input parameters of the examples and produces the corresponding meaningful output or so-called target. The adjustment process is guided by instructions on how to measure the distance between the currently produced output and the desired output. These instructions are called the objective function. At 716, the trained and tested DL/ML computational model is provided.

Fig. 7B is a simplified flow diagram showing a method 720 of inferring a type and a magnitude of a physical change to a US cable using the computational model trained according to the method 720 in accordance with a representative embodiment. Various aspects and details described above in connection with representative embodiments of Figs. 1-6A may be common to the representative embodiments described in connection with Fig. 6B. These common aspects and details may not be repeated to avoid obscuring the description. Most notably, the method 720 is contemplated for not only the method, but also for storing in a tangible, non-transitory computer-readable medium that stores instructions, which when executed by a processor, cause the processor to carry out the method of inferring (predicting) a shape of the US cable.

The computed shape is used to determine the US probe and cable use to better predict the stress level of the cable at different locations (e.g., curvature analysis etc.), the position and orientation of the US probe, the patient's surface under examination, the sonographer's movements, and other similar information indicated by the computed shape.

The method starts at 722 where motion of the US cable is detected, or forces incident on the US cable, or both are sensed. The motion may be detected via a motion sensor, an accelerometer, a vibration sensor or when forces incident on the cable are sensed through any of the sensors 204, 304, 306 and 404.

At 724, N data samples are acquired from sensors disposed over the US cable. These N data samples include data of the n-parameters of 702~706, and other parameters such publicly and privately available parameter data sets 708, 710. These data are at successive times tₙ from a US machine comprising the US cable under.

At 726, the N data samples are provided to the trained computational model to determine the type and amount of change to the US cable in order for example to determine the cable's current shape in 3D space. Notably, the computational model applied at 726 may be a trained ML or a trained and tested DL model at 716 such as described above.

At 728, the method ends with the output of results to a next user interface module for distribution and visualization and stored for further use. Again, and just by way of illustration, the output from 728 may include information on the actual shape of the US cable. The computed shape is used to determine the US probe and cable use to better predict the stress level of the cable at different locations (e.g., curvature analysis etc.), the position of the US probe, the patient's surface under examination, the sonographer's movements etc.

As will be appreciated by one of ordinary skill in the art having the benefit of the present disclosure, the systems and methods of the present teachings provide an improvement in the function of an imaging system, such as the US imaging systems described herein, and an improvement to the technical field of medical imaging. For example, compared to known methods and systems, by predicting cable integrity of the US cable based on ground truth data, or forecasting a remaining useful life (RUL) of the US cable using ground truth data, or determining an actual shape of the US cable, or a combination thereof, valuable information can be garnered. Among other benefits and practical applications, the information gathered by the application of various computational models of the present teachings, enables the avoidance of errors or breakage of the US cable because of a precarious position or undue force applied to the US cable. Moreover, being able to forecast the RUL of the US cable, and thus the ability to schedule US cable replacement rather than having to stop during an imaging session because a cable needs to be replaced affords clear benefits through a reduction of unplanned down time. Notably, the benefits are illustrative, and other advancements in the field of medical imaging will become apparent to one of ordinary skill in the art having the benefit of the present disclosure. Moreover, the methods and systems of the various representative embodiments can only be implemented on a computer given the size and complexity of the task at hand and cannot practically be performed in the human mind.

### Illustrative Applications

Below are listed two illustrative applications of the present teaching in accordance with representative embodiments. These illustrative applications are not meant to be limiting, but rather to provide examples of how aspects of the present teachings are implemented.

In a first representative embodiment the E-textile strip configuration (e.g., sensors 204) is more complex from the geometrical standpoint to capture the needed parameters to reconstruct the 3D shape of the cable; the initial phases are the same as described above in connection with Fig. 5B. A differentiating factor is an additional AI module (e.g., a deep learning model with the cable physical model embedded from 618 or 718 above) that from the obtained local strains and pressure values, calculates the *local* deformational rates, from which we finally reconstruct the overall curvilinear shape of the deformed cable. A method of reconstruction of a three-dimensional space curve is essential for the final stage of the shape sensing process. In this embodiment we use the classical Frenet-Serret formulas for the three-dimensional space curve reconstruction, i.e., to derive the *natural equation* of the 3D curve designed by the full cable length. In this process (essentially an initial value problem governed by a set of ordinary differential equations) the position, curvature, and torsion value of the initial point of the curve designed by the US cable, i.e., the cable attachment to the fixed base (i.e., cart, tablet etc.) is used as *initial condition.* The 3D curve piecewise approximation of the cable is so constructed and stored; this shape evolves in time, so the sampling rate of the reading electronics determines the update frequency of the 3D curve following the user movements. All the shapes are stored to reproduce the cable movement history. This history of the cable shapes is used by subsequent AI modules to analyze the probe position (e.g., to analyze together with the US images the quality of the acquisition, the correctness of the protocol used etc.), the sonographer's movements (e.g., to monitor if he is tired, if anti-ergonomics movement are performed, if errors are made during the procedure etc.), to reconstruct an approximated 3D surface of the user anatomy to superimpose with the acquired US images to render a more complete 3D visualization of the examined anatomy.

In a second representative embodiment, a shape of a space curve is determined by its two intrinsic quantities known as curvature and torsion; the curvature represents the rate of change of the tangential direction due to the bending of the curve, and the torsion represents the rate of change of the bending direction (i.e., the plane in which the curve section is contained). So, the ultimate goal of the E-textile component of the invention is to determine the curvature and torsion values in different positions of the US cable. The numerical method applied to the 3D curve, though, suffers from precision problems (common to the standard algorithm that uses the 4^{th} order Runge-Kutta method to solve the ordinary differential equations of the Frenet-Serret formulas) mostly related to the loss of orthonormality of the vectors that, to be recovered, require a lot of additional computation to guarantee a high spatial precision. This high spatial precision could be used, for example, to precisely localize the US probe even at the mm or sub-mm level.

To obtain such spatial precision this embodiment uses both a different geometry of the E-textile sensing element like in Fig. 3, both a different approximation of the US cable spatial curve: helicoidal approximation like in Figs. 4A, 4B. Instead of considering the cable parts between two measuring E-textile points as almost linear we consider them as approximated arcs of a helix passing through them. The innovative point of the invention is to directly put the measuring E-textile parts on the cable according to a helicoidal geometry (refer for example to Fig. 4A and 4B) so to optimize the precision of the acquired values of curvature and torsion. The flow is the same to the one in Embodiment 1 and 2 but the values are read along the E-textile strip wrapped around the cable in a helicoidal geometry. When the pressure and stretching values are acquired the 3D shape of the US cable is reconstructed by rotating the Frenet-Serret reference framework; this phase doesn't require any differential equation integration resulting in a much simpler, faster, and accurate method. The helicoidal shape of the sensing E-textile element allows a faster and more precise determination of the curve parameters. The reconstructed 3D curve in this embodiment is much more precise and can enable applications that require a very precise (mm or sub-mm precision) localization of the probe.

Although methods, systems and components for monitoring and predicting various aspects of a US cable using a computational model with reference to several exemplary embodiments have been described, it is understood that the words that have been used are words of description and illustration, rather than words of limitation. Changes may be made within the purview of the appended claims, as presently stated and as amended, without departing from the scope of interventional procedure optimization in its aspects. Although developing systems and components for monitoring and predicting various aspects of a US cable, including its shape, have been described with reference to particular means, materials and embodiments, developing systems and components for monitoring and predicting various aspects of a US cable, including its shape, is not intended to be limited to the particulars disclosed; rather developing systems and components for monitoring and predicting various aspects of a US cable, including its shape, extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive. In the claims, any reference signs placed between parentheses shall not be construed as limiting the claim.

Although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The computer program product may be software available for download from a server, e.g., via the internet. Alternatively, the computer program product may be a suitable (non-transitory) computer-readable medium on which the instructions are stored, such as an optical storage medium or a solid-state medium, which may or may not be supplied together with or as part of other hardware.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true scope of the present disclosure. Thus, to the maximum extent allowed by law, the scope of the present disclosure is to be determined by the broadest permissible interpretation of the following claims and shall not be restricted or limited by the foregoing detailed description.

## Claims

1. An ultrasound host comprising a computing device (115) comprising a controller (120) for predicting ultrasound cable integrity and/or forecasting remaining useful life of ultrasound cables, the controller being configured for:
receiving a plurality of signals from a resistive strain sensor in an ultrasound cable (112), each signal corresponding to a motion event of the ultrasound cable;
processing the plurality of signals to:
determine a strain and change of strain along the length of the ultrasound cable during each motion event; and
- predict the integrity of the ultrasound cable based on the strain and the change of strain; and/or
- forecast a remaining useful life of the ultrasound cable based on the strain and the change of strain; and
outputting the predicted integrity and/or forecasted remaining useful life.

2. The ultrasound host of claim 1, wherein the controller is further configured for any one or a combination of:
processing the plurality of signals to determine an actual three-dimensional shape of the ultrasound cable;
processing the plurality of signals to determine a force acting along the length of the ultrasound cable; and
wherein the predicted integrity and/or the forecasted remaining useful life is further based on the actual three-dimensional shape of the ultrasound cable and/or the force acting along the length of the ultrasound cable.

3. The ultrasound host of claim 2, wherein the controller is further configured to determine any one or a combination of:
a position of a clinician handling an ultrasound probe connected to one end of the ultrasound cable;
movements of the clinician operating the ultrasound probe;
a three-dimensional position of the ultrasound probe based on a three-dimensional shape of the ultrasound cable (112) and a three-dimensional position of the host connected to another end of the ultrasound cable; and
a three-dimensional surface anatomy based on positions of the ultrasound probe.

4. The ultrasound host of any one of the preceding claims, wherein the controller is further configured for:
processing the plurality of signals to determine a location of compromised integrity of the ultrasound cable, and
outputting the location of compromised integrity of the ultrasound cable.

5. The ultrasound host of any one of the preceding claims, wherein the controller is further configured for:
issuing, based on the ultrasound cable integrity and/or the remaining useful life of the ultrasound cable, a notification to replace the ultrasound cable.

6. The ultrasound host of any one of the preceding claims, wherein the processing and/or determining steps are performed by one or more trained machine learning models.

7. An ultrasound cable (112, 202, 302, 402) comprising:
a flexible textile-like substrate; and
a resistive strain sensor (204, 304, 306, 402) embedded in the substrate and distributed along a length of the cable (112); and
a communication link for connecting the resistive strain sensor to a computing device of an ultrasound host, wherein the communication link is configured to communicate measurements from the resistive strain sensor to the computing device.

8. The ultrasound cable of claim 7, wherein the resistive strain sensor comprises an electronic textile, E-textile, fabric positioned along the length of the cable.

9. The ultrasound cable of claim 8, wherein the E-textile fabric comprises a first element along the length of the ultrasound cable (112), and a second element positioned perpendicularly to the first element.

10. The ultrasound cable of claim 8, wherein the E-textile fabric is disposed helicoidally around the ultrasound cable (112).

11. The ultrasound cable of any one of claims 7 to 10, wherein the resistive strain sensor is along a substantial length of the ultrasound cable, preferably more than 50% of the total length of the ultrasound cable.

12. The ultrasound cable of any one of claims 7 to 11, further comprising a motion sensor.

13. An ultrasound system (100) comprising:
an ultrasound host according to any one of claims 1 to 6;
an ultrasound probe (110); and
an ultrasound cable (112) according to any one of claims 7 to 12 connecting the ultrasound probe with the ultrasound host.

## Patentansprüche

1. Ultraschallgerät, umfassend eine Rechenvorrichtung (115), umfassend eine Steuereinheit (120) zum Vorhersagen der Ultraschallkabelintegrität und/oder zum Prognostizieren der verbleibenden Nutzungsdauer von Ultraschallkabeln, wobei die Steuereinheit ausgebildet ist für:
Empfangen einer Vielzahl von Signalen von einem resistiven Dehnungssensor in einem Ultraschallkabel (112), wobei jedes Signal einem Bewegungsereignis des Ultraschallkabels entspricht;
Verarbeiten der Vielzahl von Signalen zum:
Bestimmen einer Dehnung und Veränderung der Dehnung entlang der Länge des Ultraschallkabels während jedes Bewegungsereignisses; und
- Vorhersagen der Integrität des Ultraschallkabels basierend auf der Dehnung und der Veränderung der Dehnung; und/oder
- Prognostizieren der verbleibenden Nutzungsdauer des Ultraschallkabels basierend auf der Dehnung und der Veränderung der Dehnung; und
Ausgeben der vorhergesagten Integrität und/oder der prognostizierten verbleibenden Nutzungsdauer.

2. Ultraschallgerät nach Anspruch 1, wobei die Steuereinheit weiter für eines oder eine Kombination von Folgenden ausgebildet ist:
Verarbeiten der Vielzahl von Signalen, um eine tatsächliche dreidimensionale Form des Ultraschallkabels zu bestimmen;
Verarbeiten der Vielzahl von Signalen, um eine entlang der Länge des Ultraschallkabels wirkende Kraft zu bestimmen; und
wobei die vorhergesagte Integrität und/oder die prognostizierte verbleibende Nutzungsdauer weiter basierend auf der tatsächlichen dreidimensionalen Form des Ultraschallkabels und/oder der entlang der Länge des Ultraschallkabels wirkenden Kraft bestimmt wird.

3. Ultraschallgerät nach Anspruch 2, wobei die Steuereinheit weiter zum Bestimmen für eines oder eine Kombination von Folgenden ausgebildet ist:
einer Position eines Klinikers, der eine an einem Ende des Ultraschallkabels angeschlossene Ultraschallsonde handhabt;
Bewegungen des Klinikers, der die Ultraschallsonde bedient;
einer dreidimensionalen Position der Ultraschallsonde basierend auf einer dreidimensionalen Form des Ultraschallkabels (112) und einer dreidimensionalen Position des an einem weiteren Ende des Ultraschallkabels angeschlossenen Patienten; und
einer dreidimensionalen Oberflächenanatomie basierend auf den Positionen der Ultraschallsonde.

4. Ultraschallgerät nach einem der vorstehenden Ansprüche, wobei die Steuereinheit weiter ausgebildet ist für:
Verarbeiten der Vielzahl von Signalen, um eine Stelle zu bestimmen, an der die Integrität des Ultraschallkabels beeinträchtigt ist, und
Ausgeben der Stelle, an der die Integrität des Ultraschallkabels beeinträchtigt ist.

5. Ultraschallgerät nach einem der vorstehenden Ansprüche, wobei die Steuereinheit weiter ausgebildet ist für:
Aussenden einer Benachrichtigung zum Austauschen des Ultraschallkabels basierend auf der Integrität des Ultraschallkabels und/oder der verbleibenden Nutzungsdauer des Ultraschallkabels.

6. Ultraschallgerät nach einem der vorstehenden Ansprüche, wobei der Verarbeitungs- und/oder Bestimmungsschritt durch ein oder mehrere trainierte Modelle des maschinellen Lernens durchgeführt werden.

7. Ultraschallkabel (112, 202, 302, 402), umfassend:
ein flexibles, textilartiges Substrat; und
einen in das Substrat eingebetteten und über die Länge des Kabels (112) verteilten resistiven Dehnungssensor (204, 304, 306, 402); und
eine Kommunikationsverbindung zum Verbinden des resistiven Dehnungssensors mit einer Rechenvorrichtung eines Ultraschallgeräts, wobei die Kommunikationsverbindung so ausgebildet ist, dass sie Messwerte vom resistiven Dehnungssensor an die Rechenvorrichtung übermittelt.

8. Ultraschallkabel nach Anspruch 7, wobei der resistive Dehnungssensor ein elektronisches Textil-, E-Textil, Gewebe umfasst, das entlang der Länge des Kabels positioniert ist.

9. Ultraschallkabel nach Anspruch 8, wobei das E-Textilgewebe ein erstes Element entlang der Länge des Ultraschallkabels (112) und ein zweites Element umfasst, das senkrecht zum ersten Element positioniert ist.

10. Ultraschallkabel nach Anspruch 8, wobei das E-Textil-Gewebe spiralförmig um das Ultraschallkabel (112) angeordnet ist.

11. Ultraschallkabel nach einem der Ansprüche 7 bis 10, wobei sich der resistive Dehnungssensor über eine wesentliche Länge des Ultraschallkabels erstreckt, vorzugsweise über mehr als 50 % der Gesamtlänge des Ultraschallkabels.

12. Ultraschallkabel nach einem der Ansprüche 7 bis 11, weiter umfassend einen Bewegungssensor.

13. Ultraschallsystem (100), umfassend:
ein Ultraschallgerät nach einem der Ansprüche 1 bis 6;
eine Ultraschallsonde (110); und
ein Ultraschallkabel (112) nach einem der Ansprüche 7 bis 12, das die Ultraschallsonde mit dem Ultraschallgerät verbindet.

## Revendications

1. Hôte pour système ultrasonore comprenant un dispositif informatique (115) comprenant un dispositif de commande (120) permettant de prédire l'intégrité du câble de système ultrasonore et/ou prévoir la durée de vie restante des câbles pour système ultrasonore, le dispositif de commande étant configuré pour :
recevoir une pluralité de signaux provenant d'un capteur de contrainte résistif dans un câble de système ultrasonore (112), chaque signal correspondant à un événement de mouvement du câble de système ultrasonore ;
traiter la pluralité de signaux pour :
déterminer la déformation et la variation de déformation le long du câble de système ultrasonore lors de chaque événement de mouvement ; et
- prédire l'intégrité du câble de système ultrasonore en fonction de la déformation et de la variation de déformation ; et/ou
- prévoir la durée de vie restante du câble de système ultrasonore en fonction de la déformation et de la variation de déformation ; et
afficher l'intégrité prédite et/ou la durée de vie restante prévue.

2. Hôte pour système ultrasonore selon la revendication 1, dans lequel le dispositif de commande est configuré en outre pour l'une quelconque ou une combinaison :
du traitement de la pluralité de signaux pour déterminer la forme tridimensionnelle réelle du câble de système ultrasonore ;
du traitement de la pluralité de signaux pour déterminer une force agissant sur la longueur du câble de système ultrasonore ; et
dans lequel l'intégrité prédite et/ou la durée de vie restante prévue est basée en outre sur la forme tridimensionnelle réelle du câble de système ultrasonore et/ou la force agissant sur la longueur du câble de système ultrasonore.

3. Hôte pour système ultrasonore selon la revendication 2, dans lequel le dispositif de commande est configuré en outre pour déterminer l'un quelconque ou une combinaison :
d'une position d'un clinicien manipulant une sonde ultrasonore reliée à une extrémité du câble de système ultrasonore ;
des mouvements du clinicien faisant fonctionner la sonde ultrasonore ;
d'une position tridimensionnelle de la sonde ultrasonore basée sur la forme tridimensionnelle du câble de système ultrasonore (112) et sur la position tridimensionnelle de l'hôte relié à l'autre extrémité du câble de système ultrasonore ; et
d'une anatomie de surface tridimensionnelle basée sur les positions de la sonde ultrasonore.

4. Hôte pour système ultrasonore selon l'une quelconque des revendications précédentes, dans lequel le dispositif de commande est configuré en outre pour :
le traitement de la pluralité de signaux afin de déterminer l'emplacement de l'intégrité compromise du câble de système ultrasonore , et
l'affichage de l'emplacement de l'intégrité compromise du câble de système ultrasonore.

5. Hôte pour système ultrasonore selon l'une quelconque des revendications précédentes, dans lequel le dispositif de commande est configuré en outre pour :
l'émission, basée sur l'intégrité du câble de système ultrasonore et/ou la durée de vie restante du câble de système ultrasonore, d'une notification pour remplacer le câble de système ultrasonore.

6. Hôte pour système ultrasonore selon l'une quelconque des revendications précédentes, dans lequel les étapes de traitement et/ou de détermination sont réalisées par un ou plusieurs modèles d'apprentissage automatique entraînés.

7. Câble de système ultrasonore (112, 202, 302, 402) comprenant :
un substrat flexible de type textile ; et
un capteur de contrainte résistif (204, 304, 306, 402) intégré dans le substrat et réparti sur toute la longueur du câble (112) ; et
une liaison de communication permettant de relier le capteur de contrainte résistif à un dispositif informatique d'un hôte pour système ultrasonore, dans laquelle la liaison de communication est configurée pour transmettre les mesures du capteur de contrainte résistif au dispositif informatique.

8. Câble de système ultrasonore selon la revendication 7, dans lequel le capteur de contrainte résistif comprend un tissu textile électronique, E-textile, positionné sur toute la longueur du câble.

9. Câble de système ultrasonore selon la revendication 8, dans lequel le tissu E-textile comprend un premier élément sur toute la longueur du câble de système ultrasonore (112) et un second élément positionné perpendiculairement au premier élément.

10. Câble de système ultrasonore selon la revendication 8, dans lequel le tissu E-textile est disposé de manière hélicoïdale autour du câble de système ultrasonore (112).

11. Câble de système ultrasonore selon l'une quelconque des revendications 7 à 10, dans lequel le capteur de contrainte résistif est situé sur une longueur importante du câble de système ultrasonore, de préférence sur plus de 50 % de la longueur totale du câble de système ultrasonore.

12. Câble de système ultrasonore selon l'une quelconque des revendications 7 à 11, comprenant en outre un capteur de mouvement.

13. Système ultrasonore (100) comprenant :
un hôte pour système ultrasonore selon l'une quelconque des revendications 1 à 6 ;
une sonde ultrasonore (110) ; et
un câble de système ultrasonore (112) selon l'une quelconque des revendications 7 à 12 reliant la sonde ultrasonore à l'hôte pour système ultrasonore.
